# EUROPEAN PATENT APPLICATION

(11) **EP 4 016 537 A1**
(43) Date of publication of application: **22.06.2022**
(21) Application number: 20000475.2
(22) Date of filing: 17.12.2020
(51) Int. Cl.: G16H 20/10, G16H 20/60

(54) **SYSTEM AND METHOD FOR BLOCKCHAIN BASED DIGITAL THERAPEUTIC DEVICES TO PREDICT FOOD-DRUG CONSTITUENT INTERACTION**

(71) Applicant: Epillo Health Systems OÜ, 11316 Tallinn, Harju maakond (EE)
(72) Inventor: Shah, Aasif, 148101 Punjab (IN); Singh, Bhupinder, Nampa, ID 83686 (US)
(74) Representative: Koitel, Raivo

(57) **Abstract**

The invention comprises systems and methods powered by an algorithm and machine learning models with the security, safety, consent sharing and smart contract features of a blockchain distributed ledger model to create a patient facing system providing a real time Food ingredient and Drug constituent Interaction detector platform and a data visualization tool to evaluate clinical outcomes generated by the systems and methods as described in this invention.

## Description

### FIELD OF INVENTION

This invention relates generally to the broader field of digital health landscape, and more specifically to a new and useful method for patient engagement in the field of healthcare called Digital Therapeutic Devices (Software based Therapeutics) used in conjunction with traditional drug plans to optimize patient care, smart engagement and tracking clinical outcomes. The present invention includes systems and methods powered by an algorithm and machine learning models with the security, safety, consent sharing and smart contract features of a blockchain distributed ledger model to create a patient facing system providing a real time Food ingredient and Drug constituent Interaction detector platform and a data visualization tool to evaluate clinical outcomes generated by the systems and methods as described in this invention.

### BACKGROUND OF THE INVENTION

As the consumption of the number of prescription drugs and OTC drugs by individuals increases over time, the possibility of DFIs (Drug Food Interactions) with unknown casual mechanisms also increases in a proportionate manner. With the evolution in the standard living of mankind in every sphere of life, the consumption of food products has also drastically evolved. The demands for packaged food, groceries, personalized meal, meals from restaurants and brands are on the rise. A trite inference would be that the horizons of the consumption have expanded considerably. For example, a consumer nowadays has the option of using mustard, marinara, ketchup, sweet onion, ketchup etc. just for the topping of the food that he eats, in short food customization and personalization have increased drastically along with food availability and consumption habits.

According to a research conducted in 2015, 47 % of the total population of United States consumes prescription drugs. Currently, the consumers of health system often receive medical notes from their doctors regarding food prescriptions, preferably through EHR (Electronic Health Recording) system which are accessible to the both the consumer and the doctor. However, such notes may not take into account the multiple number of food components that the consumer intakes from amongst the increased meal options available to him which consequentially abets the Adverse Drug Events triggered by way of drug-food component interactions.

Furthermore, another factor to be kept in mind is that the instructions provided by the doctor regarding the dietary preferences while intaking the prescribed drugs may be incomplete, inaccessible, cumbersome to understand or interpret, and not readily available to the consumer at a certain moment when he/she is making a decision on the food choices such as while ordering at a restaurant or while cooking a meal. To elaborate, A hypertension patient could be put on a restricted diet by a doctor, but, such restrictions may either not be readily available to the patient or might not account for all the multiple food components that his meal would consist of. Actionable information at the point of the decision making of the food/meal is often not provided causing the unwarranted actions of drug.

The possibility of multiple drug-food interactions may further be affected by other health parameters such as underlying chronic diseases and family history of diseases. Various other factors such as the sleeping cycle of the individual consumer, nutritional index, genetic structure, exercise levels or, sources of food constituents may further affect the working mechanism of prescription drugs in the body. To expand further, Pharmacokinetics determine the relationship between a medication administration and the concentration which it reaches in the body with time (in relationship with the dosage, presentation, frequency and form of administration), and despite the fact that many changes of these parameters have been described, it varies for each individual uniquely. Therefore, it becomes necessary to adapt the health systems with integral and multidisciplinary approaches which considers factors such as pharmacokinetic, pharmacogenetics, pharmacodynamics etc. suitable to the uniqueness of everyone's unique intake of different food components coupled with the other factors as aforementioned. Co-administration of food or drink with active prescriptions can affect drug release (volume and composition of luminal fluids, transit times, motility), absorption mechanism of the drug (uptake and efflux transporters), distribution of the drug throughout the cells (lymphatic drug transport, lipoprotein and plasma protein binding), metabolism and elimination mechanism (drug-metabolizing enzymes and drug transporters). The intake of drinks and food can change the physiology of the human GI tract and this may affect the pharmacokinetic profile of orally administered drugs ie. changing its release, absorption, distribution, metabolism and/or elimination, bioavailability, pharmacokinetics, efficacy or adverse effects. In order to influence/effect pharmacokinetic profile of a drug, the interactions must generate adducts or complexes with chemical physical properties significantly different from the original reactants. Adverse drug-food interaction episode can also lead to lower drug adherences among individuals and patients.

Data sharing in healthcare has always been a security and safety concern for individual consumers and between organizations and consumers. It is of the much interest and relevance in today's time to present a secure, safe and anonymous data transmission and storage model and therefore this invention provides that the health data of patients is stored by way of an immutable, transparent, decentralized and distributed data base in the form of blocks connected together by means of a hash. This database can be leveraged into providing a secure and trusty value chain which excludes reliance on any third party to store the patient data. The information is stored on a public ledger within blocks referenced by hash values which can be accessed by health care providers, consumers, pharmacies, food companies, research organizations etc. by way of smart contracts. In the present invention, the patient would not only have full access to his/her data which is shared with due permission but, also have the ability to audit the same by inspecting his log in the blockchain.

The present invention hence, provides the consumer with a real time Food-Rx interaction system in the form of a consumer facing application which can be used at the moment of Food/Meal decision making which aids in achieving the desired effect of a drug, avoid interactions and is personalized and updated according to the data points on health parameters, taste and nutritional requirements of the consumer, aiding with the safety, security and storage of a blockchain based undistributed ledger system.

### SUMMARY OF THE INVENTION

Drug interactions can be classified into: DDIs, DFIs, Drug-Alcohol Interactions and Drug-Disease Interactions. These interactions often result in Adverse drug events (ADEs) or reactions or reduced activity of active prescriptions. Undesirable pharmacological effects in a consumer of prescription drugs are often triggered by the drug interactions either amongst themselves or with the food that the consumer intakes. Also, effects of any drugs can be slowed down by consumption of specific foods which block their absorption capacities. A drug is administered with the intention of efficaciously providing medical aid, however, this efficacy can be altered when such drug is co-administered with another drug or consumed together with certain food components. Also, the absorption or bioavailability (Pharmacokinetic activity) of a drug can be altered when consumed together with particular food constituents. The present invention accordingly aims to comprehensively focus on Drug-Food constituent Interactions (DFI) to reduce unexpected Adverse Drug Events (ADEs), to achieve the efficacy aimed for by the drug and to maximize synergetic benefits when treating a disease.

With the consistent increase in the number of multiple drugs being prescribed to a patient for the treatment and with increased food consumption habits of the consumers, a need to devise systems and methods to detect these interactions in real time also increases proportionally so as to prevent the adverse drug events, achieve the efficacy aimed for by the drug and to maximize synergetic benefits when treating a disease.

According to a report, 67 % of elderly people in America were taking 5 or more medications on a daily basis. The consumption of the number of prescription drugs has also been found to the be highest in elderly people in the countries of Europe. In England it was found that 27 % adults were on more than 3 prescribed drugs per day and almost 50 % of the elderly were on 5 or more prescribed drugs per day. Adverse Drug Effects (ADEs) could have profound effects on the patients' quality of life, health, as well as create an increased burden on the healthcare system by readmission, decreased drug plan adherences, increased prescription length and costs. A study showed that ADEs accounted for approximately 3.5% of hospital admission. According to a research, ADEs were the cause of approximately 197,000 deaths in Europe annually

Therefore, it gives the present invention utmost usability and market product fit.

Drugs are often accompanied with certain user instructions such as expressly stating that the drug be taken with, or not taken with a certain food/drink or be taken a couple of hours before or after the meal so as to intentionally alter the adsorption or mitigate the side effects of the drug. For example: the intake of drugs with antioxidants that are naturally occurring in food is investigated to protect the side effects of mutagenic drugs like cisplatin or methotrexate. However, a problem that arises in the applicative and pragmatic world is that several patients, health care professionals and/or regulatory authorities tend to consider that the absence of such an instruction implies that there is none. The failure of inclusion of warnings/instructions in the set of guidelines ideally supposed to be accompanied by a drug may occur due to lack of data or that the product information (in Europe: SmPC/PL) is outdated. To elaborate further, a change in release of the drug in the presence of alcohol can be caused by the drug itself (e.g., solubility changes of the drug or excipients) and/or by the environment in which the drug is released (e.g., stimulation of acid secretion). With the accentuation of such issues and the fact that co-administration of a drug product with food or drink is sometimes unavoidable, A new approach to assist in real-time dosage and administration of OTC tablets and syrups, inhalers, injectable drugs can be assisted/analyzed/predicted/controlled through systems and methods as provided for in this invention with the aim of yielding the highest efficacy of the drug administered and its accompanying therapeutic benefits specifically for use in children, sensitive adults or the elderly population accordingly.

Also, balanced food constituents that do not react adversely with the active prescriptions and medication history of an individual are the backbone of a healthy lifestyle. However, most of the individuals would be callous in following a diet/restriction spelled out or communicated to them by a healthcare professional, nutritionist or a dietician. Furthermore, it would be impracticable for a healthcare professional, dietician or a nutritionist to be present always with the consumer/patient to cater to his/her real time needs and queries. Therefore, in the first embodiment of the invention, systems and method for such therapeutic applications are discussed.

Using a distributed ledger database, the present innovation will counter the existing issues of data security, safe transmission, real time data sharing and consent sharing of data through blockchain based smart contracts.

### DESCRIPTION OF THE DRAWINGS

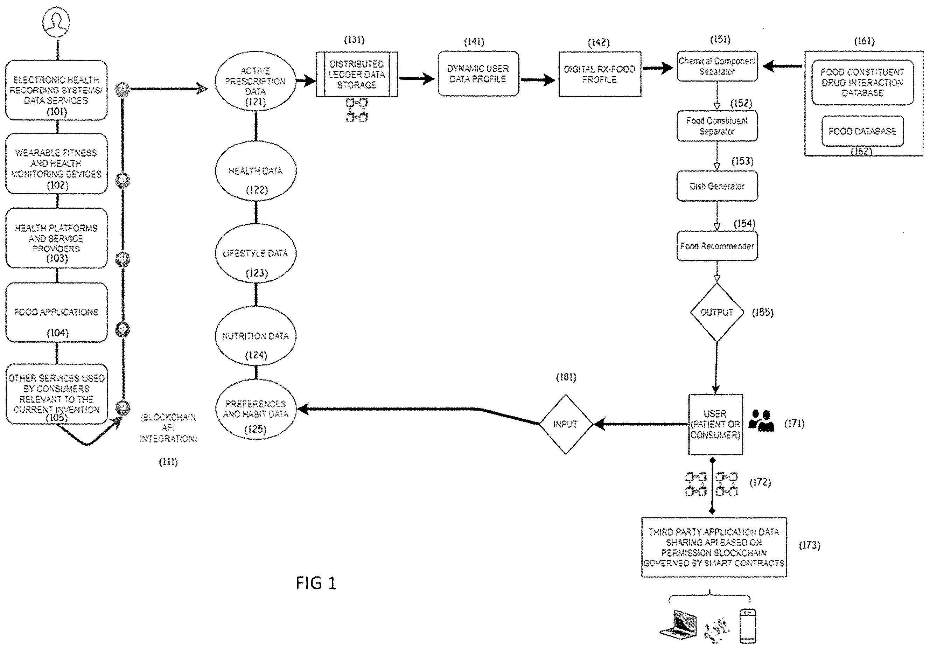

### Figure 1 INFORMATION FLOW AND DATA PROCESSING FOR USER FACING SOFTWARE BASED THERAPEUTIC SYSTEM

Figure 1 explains the flow of the working of systems and methods powered by Artificial intelligence and Machine learning models and set of algorithms to fetch the required outputs by processing, analyzing and structuring input data from various credible sources while keeping the data storage and sharing on a blockchain based distributed ledger model.
Figure 2 DRUG BINDING AND FOOD INGREDIENT DRUG CONSTITUENT INTERACTION DETECTION METHOD

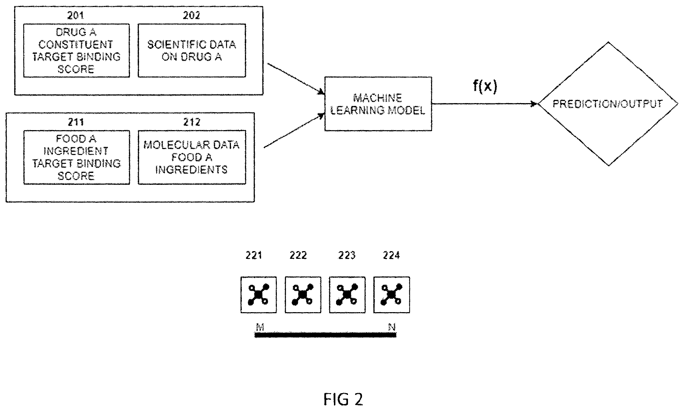

Figure 2 explains the Artificial Intelligence based molecular docking model wherein the binding scores of a Drug A (201) received from Molecular Docking platforms such as Auto Dock Vina on target protein sites 221, 222, 223, and 224 respectively is compared with the binding score of Food Ingredient (211) with the same target protein sites passed through an algorithm/function f(x) along with the existing datasets of Scientific data on Drug A (202) and molecular data of Food A ingredients (212) to predict Pharmacological changes triggered by such co-administration of Drug A and Food A.

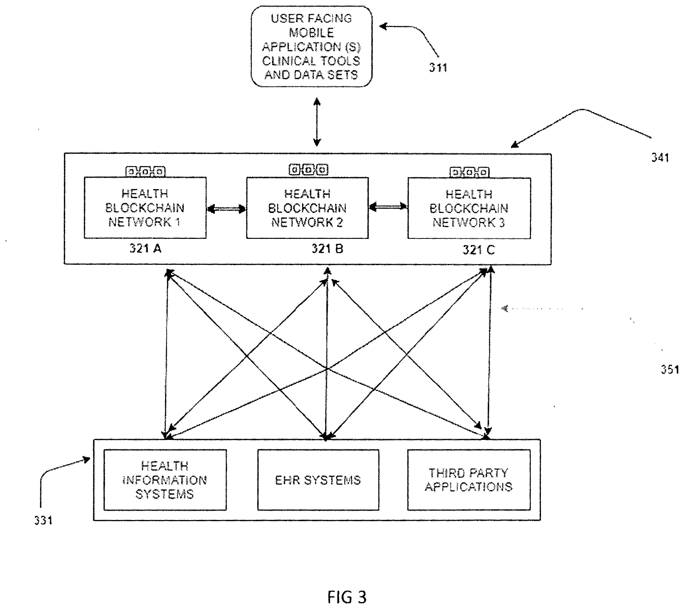

Figure 3 BLOCKCHAIN BASED HEALTH DATA EXCHANGE FRAMEWORK
Figure 3 explains the user facing mobile application, clinical tools and data sets as discussed in the present invention (311) accessing a backend decentralized blockchain ledger (341) wherein the backend system 341 may have plural health block chain network components 321 A, 321 B, 321 C and the one or more applications access single or plural health block chain network components.. Each application 311 is able to connect to and interact with the backend 341 over the communications path 351 using blockchain based smart contracts, blockchain data sharing protocols and APIs to communicate with third party applications and external applications 331.

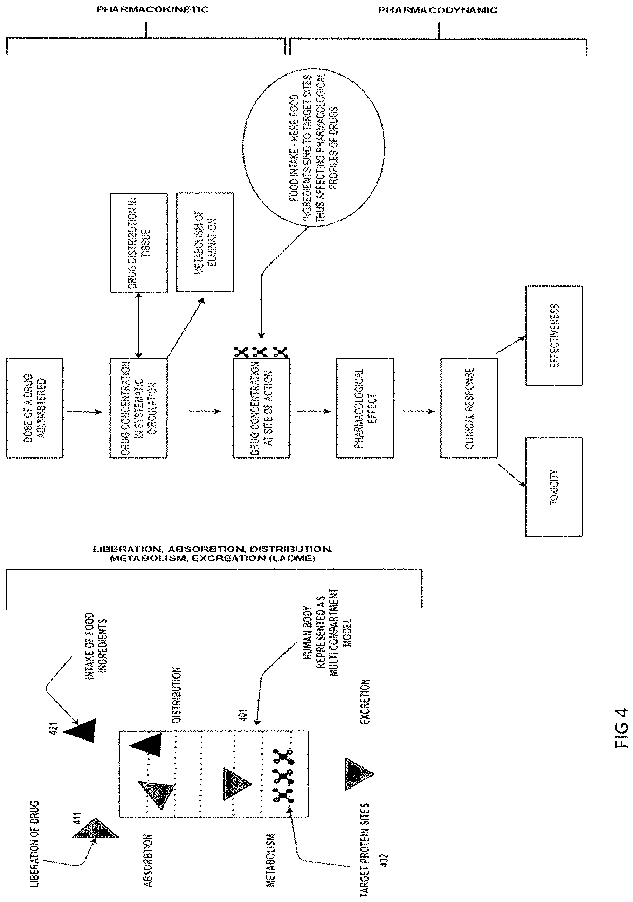

Figure 4 LIBERATION, ABSORBTION, DISTRIBUTION, METABOLISM AND EXCREATION ALONG WITH CO-ADMINISTRATION OF FOOD
Figure 4 explains Liberation, Absorption, Distribution, Metabolism, Excretion along with co-administration of food ingredients. 401 represents human body through a multi-compartment pharmacokinetic model, 411 refers to Liberation of drug, 421 refers to the intake of food ingredients, 432 is the target protein sites.

### DETAILED DESCRIPTION OF THE INVENTION

Co-administration of food or drink with active prescriptions can affect drug release (volume and composition of luminal fluids, transit times, motility), absorption mechanism of the drug (uptake and efflux transporters), distribution of the drug throughout the cells (lymphatic drug transport, lipoprotein and plasma protein binding), metabolism and elimination mechanism (drug-metabolizing enzymes and drug transporters). The intake of drinks and food can change the physiology of the human Gl tract and this may affect the pharmacokinetic profile of orally administered drugs i.e. changing its release, absorption, distribution, metabolism and/or elimination, bioavailability, pharmacokinetics, efficacy or adverse effects.

It would be impossible for a human expert such as a clinician, healthcare provider or dietician/nutritionist to follow a person/patient round the clock. Such an expert would be unable to keep track of several factors such as the individual's entire medical history (including history or data from different healthcare providers), genetic data, physical activity, non-prescription treatments, food/meal decisions, etc. unless the expert meets the individual on multiple occasions every day. Furthermore, human experts tend to align their judgements based on some specific set of rules and may, on some occasions ignore several factors which results in generic food-drug interaction guidelines.

In one embodiment of the present invention, a user facing application based on Machine learning models to evaluate and predict Food - Drug Constituents interaction through an Artificial Intelligence based computational molecular docking model has been established.

A user facing application supplemented with consumer's active prescriptions, health history, nutritional profile, eating patterns, taste profile (parameters), based on systems discussed in the present invention, would act as personalized, secure and all-time available food-Drug interaction indicator, intake recommender, meal planner and Food Menu filter. This invention provides such an assistance to the Healthcare Industry, Pharmaceutical Industry, Food industry and Consumers/Patients/People on active prescriptions while also giving the application a dynamic nature which would take into account the user feedback and on the go changes in their preferences aided by a machine learning model.

As described in Figure 1 (INFORMATION FLOW AND DATA PROCESSING FOR USER FACING SOFTWARE BASED THERAPEUTIC SYSTEM) the system would take into account various data points including but not limited to the Active prescription data, health data, lifestyle data, nutritional data of a user and another sets of data including but not limited to scientific data sets of various food constituents and drug constituents, Scientific Modules on Drug-Food Interactions, Food Literature. A mechanism/method is provided to evaluate profile of the users by way of a Machine learning model that interacts user profiles with global/scientific modules and molecular docking models to generate results. This method would also include analyzing a single user by administering multiple statistical techniques to enable the algorithm to infer user preferences and consistently update them. This data is then congregated and graded into different groups and compared using the statistical principles which provide a comprehensive set of up to date data.

The components of the algorithm enabling a Food Rx profile are: -
a) Dynamic user data stream and profile
b) Dynamic Rx Food Profile
c) Food chemical components and interaction database
d) Meal Planner
e) Machine learning method to update user habits and preferences

The various clustering methods that would apply the components of the algorithm are as follows:
1. Collaborative Filtering
2. Nearest Neighbor
3. Matrix Factorization
4. Bayesian Network
5. Control Circuitry

The different components of the algorithm working under the aegis of the clustering methods provide for a framework of drug and food interactions by utilizing their structural information as inputs processed with unique individual consumer/patient profiles to accurately generate important interactions as outputs ready to be used by a consumer/patient in the form of a clinical-like advise at the point of decision making. These predict the effects of certain food constituents on pharmacological profile of interacting drugs and bioactivities of different food constituents and informs the consumers/patients in real time to align with the advice and increase their therapeutic benefits for active drug plans.

Interactions between food constituents and drugs are evaluated through the systems and methods given in this invention on a chemical structural information/data level using an Artificial Intelligence based computational molecular docking model. The output sentences or results displayed to users or consumers are generated from the predefined important general structures representing interaction types that are prepared by an algorithm or can be translated or displayed as user understandable output sentences referred to as near clinical advice.

Analogous to drug products, food is also a mixture of different chemical entities endowed with a determinable structure and specific (re)active moieties. Nonetheless, it is clear that food is a very complex chemical system, in which low and high molecular weight components are mixed together and all of them can, in principle, cause specific food-drug interactions that can be classified in terms of binding properties.

For Example: CYP3A4 is often considered the most important drug-metabolizing enzyme, given its relatively high expression in liver and intestine. Pre-administration of an inhibitor of CYP3A4 enzyme (through a food constituent as in the case of current invention) alter the pharmacokinetics or specifically the release mechanism of a drug which binds to CYP3A4 enzymes. Depending on the working mechanism of the drug and CYP3A4 enzyme, this interaction can either elevate concentrations of an active drug in the body or lower concentrations of the active drug, thus affecting absorption, distribution, bioavailability and excretion mechanism of a drug (pharmacokinetic profile). In order to have an effect on pharmacokinetic profile of a drug, the interactions must generate adducts or complexes with chemical physical properties significantly different from the original reactants.

Pharmacodynamic food-drug interactions are caused by a specific interaction between a drug and a component of the food that results in a particular pharmacological change in the body at the binding sites. Most of the Pharmacodynamic interactions involve drug metabolizing enzymes and transport proteins. Pharmacodynamic drug-food interactions can take several forms and can lead to either enhanced activity (synergism) or decreased activity (antagonism). The most prominent example of a specific pharmacokinetic food-drug effect is the interaction between grapefruit juice and drugs like cyclosporine and felodipine. The interaction can result through different mechanisms, including the inhibition of CYP3A4 metabolism.

Medicines/Prescription drugs also contain ingredients other than the active drug that are essential for their manufacture, stability and function. These ingredients should be inert (nonreactive) however they do have the potential to cause adverse effects in sensitive individuals. For example: Our predictive framework can elucidate biological effects of commonly consumed chemical matter with implications on food- and excipient-drug interactions. Retinyl palmitate, or vitamin A palmitate, (the ester of retinol and palmitic acid) is commonly used drug-excipient in many Over the Counter (OTC) and prescription drugs and has been identified as inhibitors UGT2B7 enzyme in a scientific research.

Furthermore, Artificial Intelligence based computational molecular docking model used in the present invention can autonomously explores multiple possible binding sites and modes and score them according to the interaction potential of drug compounds/food constituent compounds/drug excipient compounds with enzymes in different target sites and detect the drug/drug excipient-food interaction type by calculating the most probable binding sites.

As illustrated in Figure 1, Data from various credible sources such as but not limited to Electronic Health Recording systems or health data aggregators (101), IOT enabled Wearable Fitness and Health Monitoring devices (102) such as Fitness Bands, Blood Pressure Monitors, Glucose Monitors, Personal Weighing Machines, etc. , Health platforms and service providers (103) such as Online Pharmacies, Pathology labs, Nutritional supplements services, Wellness products services, etc. , Food applications (104), Other services used by consumers (105) which can provide relevant input data to set heuristics for consumer health preferences applicable in the current invention.

This unstructured data stream is accessed by our system through Blockchain based API data sharing protocol (111) and structured into various dynamic labels/tags/parameters such as but not limited to Active Prescription data (121), Health Data (122), Lifestyle related data (123), Nutrition related data (124), Preferences and habits data (125) hosted on a Blockchain based Distributed ledger data storage (131) further run through a set of hyper-heuristic algorithms based on a Machine learning model to create a Dynamic User data profile (141) and Digital Rx-Food Profile (142) which is fed into the Chemical component separator (151) simultaneously fed with larger set of updated Food Constituent and Drug Interaction dataset (162) comprising of a subset - Food Database (162). The Chemical Component separator (151) processes all the data into a Food Constituent Separator (152), Dish Generator (153) and Food Recommender (154) to produce Output (155) for a User (171), further shared to Third party applications including but not limited to API or Dataset integration (173) through secure and transparent Permission Blockchain Network (172) while updating the user habits and preferences as a dynamic input (181) into the structured label/parameter of Preference and Habit data (125) to continuously feed the recommendation algorithms based on machine learning model.

In the present invention, to establish credible correlation and relationship between input data sources, various advanced machines learning, and predictive analytics models including but not limited to Linear and Mechanistic correlation models to produce recommendations and outputs as desired, are used.

Deficiency in data can compromise the algorithm results. Therefore, Strategies for data sanitation, data inclusiveness, and proper mechanisms for data correction will help further in realizing the potential of the current innovation. For better classification of minority groups, one can apply a neighborhood-based data cleaning method to better balance the classes and predict a clearer class boundary by eliminating reductant or dominant data points of the major class. The integrity of unbiased, credible and true data depends upon the reliability of input sources such as EHR systems, wearable devices and Health Monitoring devices and other third-party applications used by a consumer and transmission relies on fidelity of the database and network. Using a blockchain distributed ledger database, the present innovation will counter the existing issues of health data, security, safe transmission, real time data sharing with safety and consent through blockchain based smart contracts.

The health data of patients is stored by way of an immutable, transparent, decentralized and distributed data base in the forms of blocks connected by means of a hash. This database can be leveraged into providing a secure and trusty value chain which excludes reliance on any third party to store the patient data. The information is stored on a public ledger within blocks referenced by hash values which can be accessed by health care providers, consumers, pharmacies, food companies, research organizations etc. by way of smart contracts. In the present invention, the patient would not only have full access to his/her data which is shared with due permission but, also have the ability to audit the same by inspecting his log in the blockchain.

Furthermore, this information sharing of certain data points is assisted by an automated self-executing program deployed on blockchain. This automated system will execute predefined terms set by users in the secure container automatically by sending the contract invoking transaction to the relevant contract address. By the usage of Blockchain, the patient data can be segregated easily and only necessary data points allowing the analyst to address the specific health concern are extracted from the global set so as to develop a higher level of trust and confidentiality with the consumer.

This invention affords continuous availability of generated and processed real-time Food-Drug Interaction profile (clinical data) to Healthcare providers, Clinicians, Dieticians/Nutritionists through Blockchain based API. The distributed ledger method used to store patient/consumer data allows seamless integration with any third-party applications while providing a secure peer to peer autonomous accurate health information exchange and interoperability.

The systems and methods for the user facing digital therapeutic aid as laid in this invention provides for solution offerings such as data integration, mobile application API integration, device integrations/device hub and Solution as a service for consumers (SaaS).

Data integration solutions can be used for disease management at public health level, integrated into physicians/doctor's existing EHR workflow as an additional tool, weight nutrition and metabolism management third party applications, Health Monitoring devices such as Blood Pressure Monitors, Glucose Monitors and their connected applications, Digital Pill Bottles, Smart Fridge and Smart Microwaves. The data analytics generated by systems and methods in the present inventions can also be used by Food trend identification companies and for new balanced food product offerings in the marketplace. Device Integration can include integrating the systems and methods into physical devices at the premises of a food service environment.

The present invention envisages a future application in which an individual/patient can order, collect in-person, or consume the right meal, groceries, food items and food ingredients from any restaurant, third party food application, meal planning service, grocery provider, readymade/instant meal provider or any food busines provider relying on systems and methods provided in the current invention while keeping the doctor, physician, dietician, nutritionist and his/her loved ones (concerned family/friends) on the same blockchain distributed ledger model, updating them in real time about the clinical-like advise generated and adhered along with the consumer/patient's consequent decisions and the analytics therefrom supporting a lifetime well-being.

These balanced food recommendations and Interaction systems as explained in the present invention can be integrated with blockchain based food traceability systems to enhance consumer satisfaction and knowledge about food safety and can further be integrated with smart cooking appliances and Smart Fridges to automate the whole process of food consumption at home working in conjugation with IOT smart cooking devices.

### CHANGES IN PHARMACOLOGICAL PROFILES OF DRUGS WHEN CO-ADMINISTERED WITH FOOD

Pharmacokinetics determine the relationship between a medication administration and the concentration which it reaches in the body with time (in relationship with the dosage, presentation, frequency and form of administration).

Co-administration of food or drink with active prescriptions can affect absorption, distribution, metabolism, and excretion (ADME) Mechanism of a drug, i.e. drug release (volume and composition of luminal fluids, transit times, motility), absorption mechanism of the drug (uptake and efflux transporters), distribution of the drug throughout the cells (lymphatic drug transport, lipoprotein and plasma protein binding), metabolism and elimination mechanism (drug-metabolizing enzymes and drug transporters).

In another embodiment of the present invention, A Support Vector Machine learning Model (SVM) is used to determine the Drug concentration vs. time graph and compared to Typical Drug Screening Data available in Drug Scientific Modules to calculate efficacy of the drug and indicate changes in pharmacokinetic profile of Orally Administered and IV drugs in response to intake of different food items/ingredients based on Multi Compartment Pharmacokinetic Models.

Pharmacodynamic food-drug interactions are caused by a specific interaction between a drug and a component of the food that results in a particular pharmacological change in the body at the binding sites. Most of the Pharmacodynamic interactions involve drug metabolizing enzymes and transport proteins. Pharmacodynamic drug-food interactions can take several forms and can lead to either enhanced activity (synergism) or decreased activity (antagonism).

In another embodiment of the present invention, a support vector Machine learning Model (SVM) is used to evaluate changes in pharmacodynamic profile of Orally Administered and IV drug in comparison to the Drug's Initial Screening Data available in Scientific Modules. Interactions at drug binding sites are predicted and analyzed by computational Molecular Docking Model as discussed in embodiments of this present invention.

Concentration of a drug at the site of action controls its affects and concentration of a drug in the body in relation to time affects the efficacy of the drug. Pharmacokinetic Plasma Concentration v. Time (Dynamic Graph) and Pharmacodynamic Drug Dose - Response relationship curve formed by Machine learning models as discussed in the present invention forms the basis of evaluation of optimal drug-dosing, optimal frequency and actionable clinical data.

## Claims

1. System for blockchain based digital therapeutic devices to predict food-drug constituent interaction comprising of:
(a) Set of Algorithms to collect, process, cluster, structure and analyze User input data,
(b) Set of Algorithms to collect, process, cluster, structure and analyze Scientific Modules of Drug, Food data and associated literature,
(c) Set of Algorithms to establish credible correlation and relationship between input data sources by various advanced machines learning models to fetch the required outputs by processing, analyzing and structuring input data,
(d) Set of Algorithms to interact the user data with Drug-Food Scientific modules and Literature,
(e) Machine learning models to predict Drug-Food Interactions and learn user preferences and habits.

2. System according to claim 1, wherein Interactions between food constituents and drug constituents are evaluated through the systems and methods given in this invention on a chemical, molecular or structural information/data level using an Artificial Intelligence based computational molecular docking model which provide for a framework of drug and food interactions by utilizing their structural information as inputs processed with unique individual consumer/patient profiles to accurately generate important interactions.

3. Use of the system in Claim 1 and Claim 2 to predict drug-food constituent interactions (DFI) to reduce unexpected Adverse Drug Events (ADEs), to achieve the efficacy aimed for by the drug and to maximize or increase the therapeutic benefits of an ongoing drug plan.

4. Use of the system in Claim 1 and Claim 2 to create a user facing computer or mobile application providing real-time Drug-Food Constituent Interaction system and acting as a Digital therapeutic aid to advise on co-administration of drug and food, real time personalized food recommendation, food filtering, and intelligent grocery/meal recommendations solution/platform for Patients and their healthcare providers.

5. Method for blockchain based digital therapeutic devices to predict food-drug constituent interaction, comprising of:
(a) Obtaining from various sources: User Data - including but not limited to Active Prescription data, Health data, Lifestyle Data, Nutritional Data, Eating Patterns,
(b) Obtaining, from various databases: Drug and Food Data - including but not limited to Scientific Module on Drugs, Food Literature, Molecular Information of Prescription and Non-Prescription Drugs and their constituents, Molecular structure of the food ingredients,
(c) Predicting at the required time of decision making: A possible interaction between a food ingredient and Drug Constituent at a target protein site,
(d) Generating, at the required time of decision making: A balanced food choice satisfying a plurality of factors including but not limited to Active Prescription data, Health Data, Lifestyle Data, Eating Patterns.

6. Method according to Claim 5, wherein the plurality of User Data to be processed comprises of but not limited to: (a) Active and Inactive Prescription Data (b) Health Data, Medical Data and Disease history (c) Lifestyle Data, (d) Nutritional data profile, (f) Eating patterns, (g) Taste profile (h) Any relevant data comprising or defining Food and Health parameters of an Individual

7. Method according to Claim 5, wherein the plurality of Scientific Data streams includes but are not limited to: (a) Scientific Module on Drugs and Screening data (b) Drug Excipient Data (c) Molecular/Structural Information of Prescription and Non-Prescription Drugs and their constituents (d) Molecular structure of the food ingredients, (e) Any relevant dataset including molecular/structural, labelling, safety information of Food and Drugs.

8. Method, by using system according to Claim 1 and Claim 2 wherein the system is integrated into third party services including but not limited to: Food ordering applications, Meal planning services for schools, offices, events, restaurants, readymade/instant meal provider, Grocery service providers, Retail and packaged food product providers, Digital Menu/QR code-based Menu of Food/Meal and Beverage Services, Smart Cooking appliances and Smart Fridge by way of us established in Claim 4.

9. System and use according to Claim 1, Claim 2 , and Claim 4, wherein a blockchain based distributed and decentralized ledger model in the form of blocks connected together by means of hash is used as an immutable data storage and transmission model by the way of blockchain based smart contracts between the stakeholders.

10. System and use according to Claim 9 wherein a cryptographic hash function token is used for smart contracts.

11. Use according to Claim 4, using systems established in Claim 1 and Claim 2, wherein the analytical data generated by the application is used to increase the scientific literature on Food-Drug Interactions, Food trend identification , Adverse Drug Event (ADE) management at public health level, integrated into physicians/doctor's in existing EHR systems workflow as an additional valuable information tool, Integrated into weight, nutrition and metabolism management services, devices and solutions by way of integrations and systems established in this invention.

12. Method according to system established in Claim 1 and Claim 2, wherein a Support Vector Machine learning Model (SVM) is used to determine, evaluate and quantify the Drug concentration at different time intervals compared to Typical Drug Screening Data available in Drug Scientific Modules to calculate efficacy of the drug and indicate changes in pharmacokinetic profile of Orally Administered and IV drugs in response to intake/co-administration of different food items/ingredients based on Multi Compartment Pharmacokinetic Models, graphically and visually through visualization tools.

13. Method according to system established in Claim 1 and Claim 2, wherein a Support Vector Machine learning Model (SVM) is used to determine, evaluate and quantify changes in pharmacodynamic profile of Orally Administered and IV drug in when co-administered with food ingredients and compared to the Drug's Initial Screening Data available in Scientific Modules to establish Pharmacodynamic profile changes in the Drug mechanism in response to intake/co-administration of different food items/ingredients through computational molecular docking models, graphically and visually through visualization tools.

14. Method according to Claim 12 and Claim 13, using system according to Claim 1 and Claim 2 is established to provide dynamic drug dosing information for Orally Administered and IV drugs for Patients/Consumers.

15. Method according to Claim 14 is established using systems in Claim 1 and Claim 2 and analytical data from methods mentioned in Claim 12 and Claim 13 to be integrated with Smart Pill Bottles and Drug Dosing Information modules and existing clinical workflows for Healthcare Practitioners and Patients for calculating real-time drug dosing information systems.
